# EUROPEAN PATENT SPECIFICATION

(11) **EP 0 383 808 B1**
(45) Date of publication and mention of the grant of the patent: **07.06.1995**
(21) Application number: 88909340.7
(22) Date of filing: 31.10.1988
(51) Int. Cl.: C12N 15/00, C12P 21/02

(54) **A GENE CODING FOR GLUCURONIDE PERMEASE**
EIN GEN, DAS FÜR GLUKURONIDPERMEAS KODIERT
GENE CODANT POUR LA GLUCURONIDE PERMEASE

(30) Priority: 29.10.1987 GB 8725402
(43) Date of publication of application: 29.08.1990
(73) Proprietor: JEFFERSON, Richard Anthony, Canberra ACT 2601 (AU)
(72) Inventor: JEFFERSON, Richard Anthony, Canberra ACT 2601 (AU)
(74) Representative: Matthews, Heather Clare
(86) International application number: GB8800936
(87) International publication number: WO8903880

(56) References cited:
- GB-A- 2 197 653
- Proc. Natl. Acad. Sci. vol. 83, 1986 (USA) Richard A. Jefferson et al.
- The EMBO Journal, vol. 6, no. 13, 1987 Richard A. Jefferson et al.
- J. Mol. Biol. vol. 1987, Richard A. Jefferson et al.

## Description

### 1. FIELD OF THE INVENTION

The present invention relates to the transport protein glucuronide permease and its use for selectively altering the permeability of live cells. This transport protein can be used to facilitate the cellular uptake of various compounds conjugated to β-glucuronides, including chemical markers and substances which alter cellular metabolism.

### 2. BACKGROUND OF THE INVENTION

### 2.1 β-GLUCURONIDES

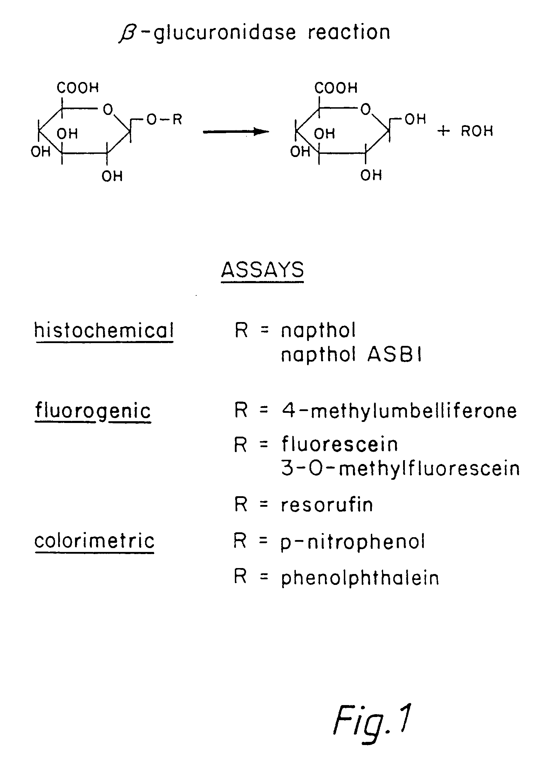

β -Glucuronides consist of glucuronic acid having various substituent groups in a hemi-acetal glycosidic linkage (Figure 1). In mammals, glucuronidation is a principle means of detoxifying or inactivatiing compounds, utilizing the glucuronyl transferase system. In humans, a number of hormones, including cortisol and aldosterone, antibiotics such as chloramphenicol, toxins such as dinitrophenol, and bilirubin are among the numerous compounds which are conjugated to glucuronides by the glucuronyl transferase system and then excreted in urine or into the lower intestine in bile. The bacterium Escherichia coli has evolved to survive in the mammalian intestine, and can utilize the excreted β -glucuronides as its sole carbon source To do so, E. coli has evolved mechanisms for the uptake and degradation of a wide variety of glucuronides, processes which are tightly linked genetically.

### 2.2 β-GLUCURONIDASE

β-Glucuronidase (GUS) is an enzyme which exhibits acid hydrolase activity, which cleaves the glycoside linkage between a glucuronide and its substituent. GUS has been characterized extensively in E. coli bacteria, as a very stable protein with a subunit weight of 68.2 KDa encoded by the uid A (gus A) genetic locus. It catalyzes the cleavage of a wide variety of glucuronides, most of which are water soluble. As illustrated by Figure 1, a variety of compounds, including, but not limited to, histochemical, fluorogenic or colorimetric markers can be conjugated to glucuronic acid to form the β-glucuronide. These β-glucuronide chemical markers can be used as indicator substrates for the GUS enzyme. Therefore, activity levels of GUS can be determined using sensitive colorimetric, fluorogenic, or histochemical detection methods.

Many organisms express little or no β-glucuronidase, including higher plants such as potato and rice, the slime mold Dictyostelium discoideum, the yeast Saccharomyces cerevisiae, and the fruitfly Drosophila melanogaster.

### 2.3. β-GLUCURONIDASE FROM E. COLI AS A GENE FUSION MARKER

The β-glucuronidase structural gene was separated from its promoter/operator and Shine Delgarno (ribosome binding) sequences, fused with the E. coli lac Z promoter sequence, and inserted into a plasmid vector by standard cloning techniques. The resulting construct, which places the β-glucuronidase gene under the control of the lac Z promoter, could be transfected into E. coli and resulted in high levels of β-glucuronidase expression (Jefferson et al., 1986, Proc. Natl. Acad. Sci. U.S.A. 83:8447-8451 and see also UK specification No. 2197653) and US 5268463. If the lac Z promoter were active in initiating transcription, the β-glucuronidase gene was expressed.

To test whether expression of cloned β-glucuronidase could be controlled by other types of promoter sequences, the β-glucuronidase gene was coupled to various transcriptional promoters. Gene fusion between β-glucuronidase and either the Cauliflower mosiac virus (CaMV) 35S promoter or the ribulose bisphosphate carboxylase promoter were used to transform tobacco plants and achieved expression of β-glucuronidase with what appeared to be promoter controlled tissue specificity (Jefferson et al., 1987, EMBO J. 6:3901-3907). Similar experiments, using mutants of the nematode Caenorhabitis elegans which do not express β-glucuronidase, fused flanking regions of the col-1 collagen gene or of a major sperm protein to the β-glucuronidase gene, resulting in expression of a β-glucuronidase fusion protein; (Jefferson, et al., 1987, J. Mol. Biol. 193:41-46). Importantly, the β-glucuronidase fusion protein showed functional enzymatic activity in organisms as diverse as bacteria, higher plants, and lower animals. Further, GUS activity could be detected in individual cells using histologic techniques.

These experiments used β-glucuronidase expression as an indicator of promoter activity. β-Glucuronidase served as a gene fusion marker, or "reporter gene", the expression of which was evaluated using a β-glucuronide indicator substrate.

In E. coli, the same genetic locus encodes β-glucuronidase and transport proteins which facilitate the uptake of β-glucuronide substrates. The isolated β-glucuronidase gene utilized in the gene fusion experiments described supra does not control the transport of β-glucuronide indicator substrates, so that measurements of β-glucuronidase activity were performed using tissue extracts or histologic sections.

### 2.4. GLUCURONIDE PERMEASE

The phospholipid bilayer membrane has evolved to selectively retain molecules within cells, and prevent the promiscuous exchange of cellular contents with the extracellular milieu. Cells have, however, developed the capacity to transport selected compounds across the permeability barrier of the membrane, even against a concentration gradient. In general, without a specific transport mechanism, polar molecules are excluded from passing across a cell membrane. Because β-glucuronides are intrinsically polar molecules, bearing a negatively-charged ionized carboxyl group, these molecules are highly impermeant to cells, and require glucuronide permease for transport.

### 3. SUMMARY OF THE INVENTION

The present invention relates to the gone encoding the transport protein, glucuronide permease. Recombinant vectors encoding glucuronide permease can be used to transfect host cells. Expression of glucuronide permease by the transformants allows cellular uptake of β-glucuronides. This system permits the detection of β-glucuronidase activity in vivo, and can conveniently be used together with GUS gene fusions.

In addition to providing an important complement to the GUS system, glucuronide permease also can be used in a method to selectively alter the permeability of cells. Because of the variety of substances which can be conjugated to β-glucuronides, glucuronide permease provides a cellular entrance for a multitude of compounds.

### 4. DESCRIPTION OF THE FIGURES

FIG. 1 illustrates the reaction catalyzed by β-glucuronidase, and the structure of substrates transported by glucuronide permease.

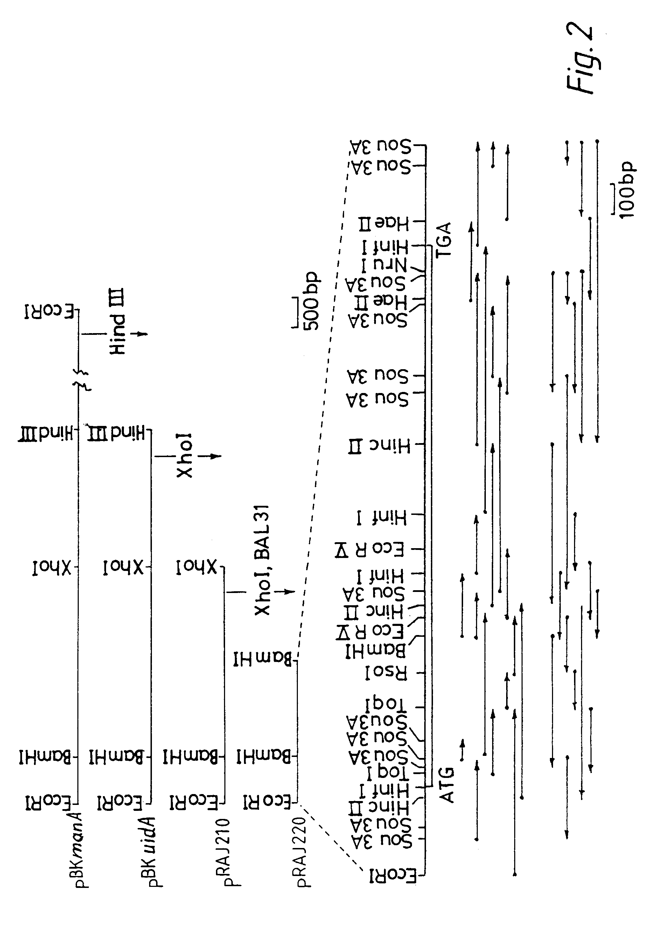

FIG. 2 illustrates the structure of various plasmids. The subcloning of the 3' end of pRAJ210, encoding the glucuronide permease, was done by cleaving pRAJ210 with Pst I (cleaving in the polylinker site of pUC9, proximal to the Xho I site of pRAJ210 and Nsi I which cleaves just 5' of the BamHI site of pRAJ220. The sequence of this region was determined by the method of Maxam and Gilbert.

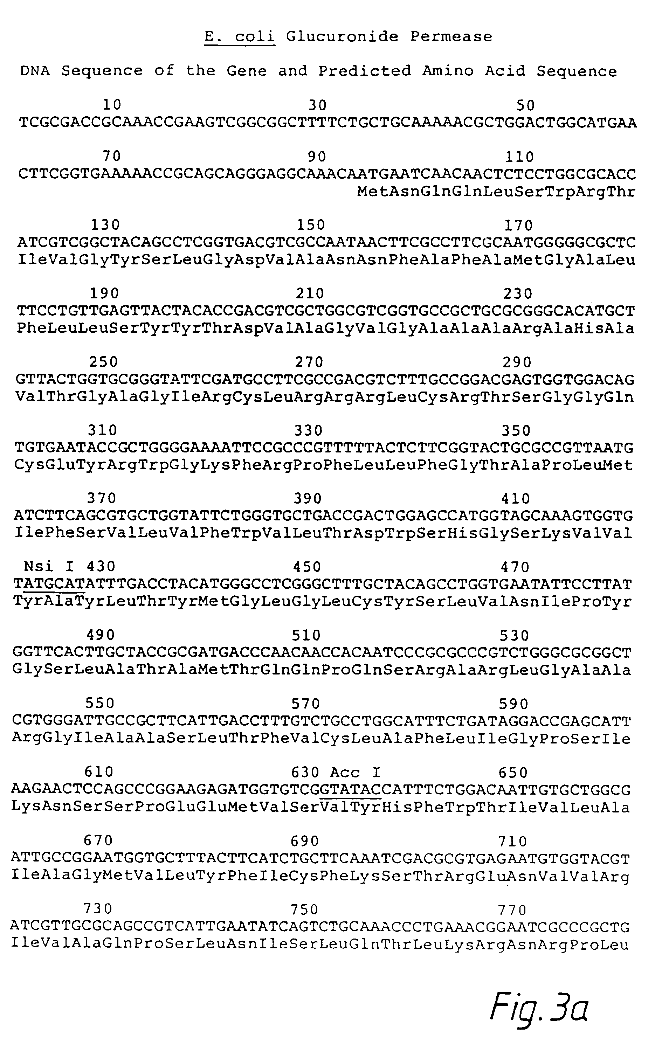

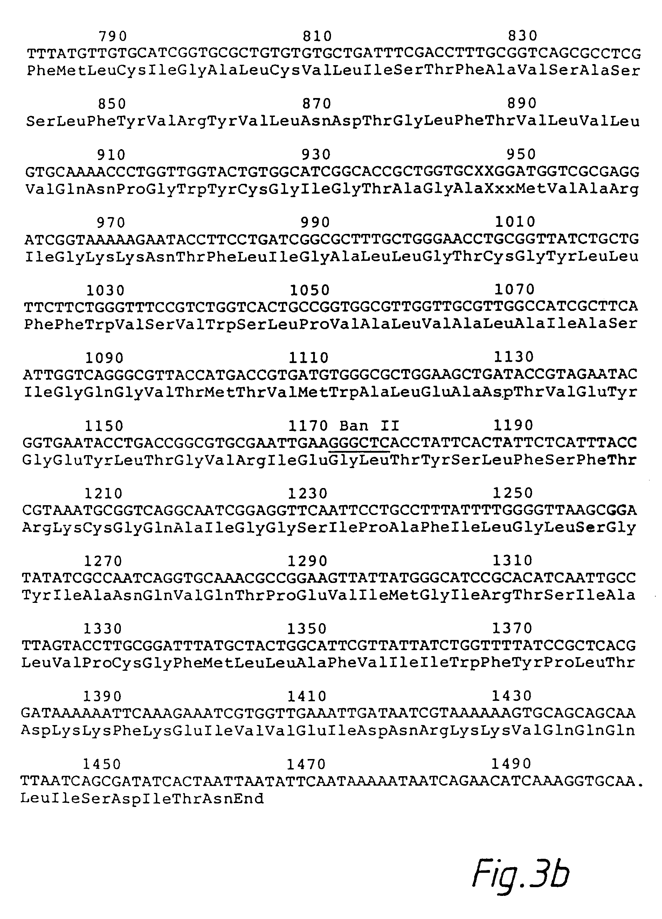

FIG. 3 illustrates the DNA sequence of the glucuronide permease gene on pRAJ285. The sequence was determined as described in the legend to FIG. 2. The sequence shown extends from the Nru I site within GUS (sequenced by dideoxy method) through the Nsi I site into the previously unsequenced region of pRAJ210. The sequence shown extends only just past the terminator codon of the permease. This is represented on the plasmid pRAJ285. The rest of pRAJ210 has been sequenced, and is present on pRAJ280 - pRAJ284.

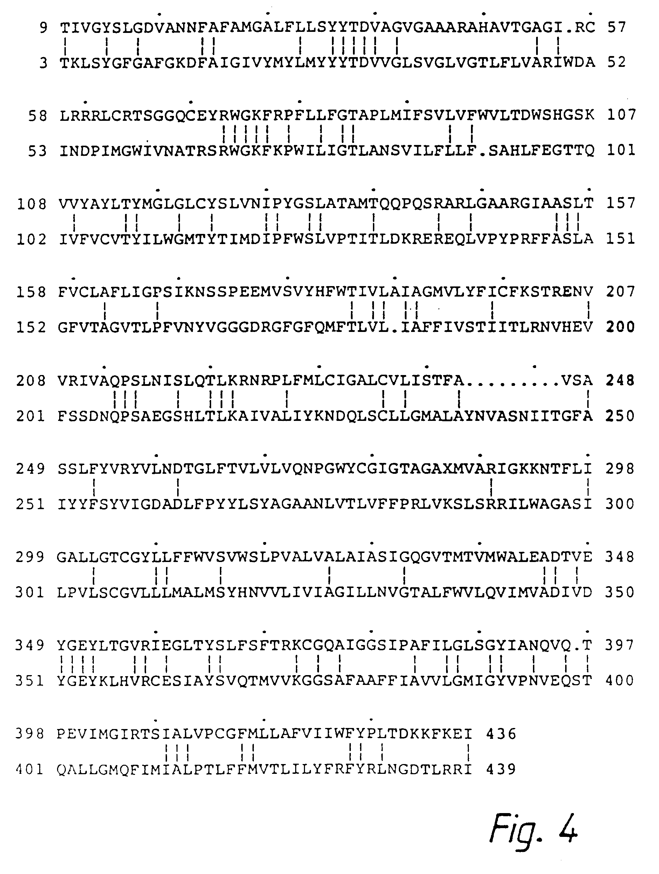

FIG. 4 is a comparison of the amino acid sequences of glucuronide permease (top) and the melibiose permease (bottom) using the University of Wisconsin Genetics Computer Group Compare programs. The lines between sequences indicate exact matches between the two sequences. Small gaps were introduced to maximize homology.

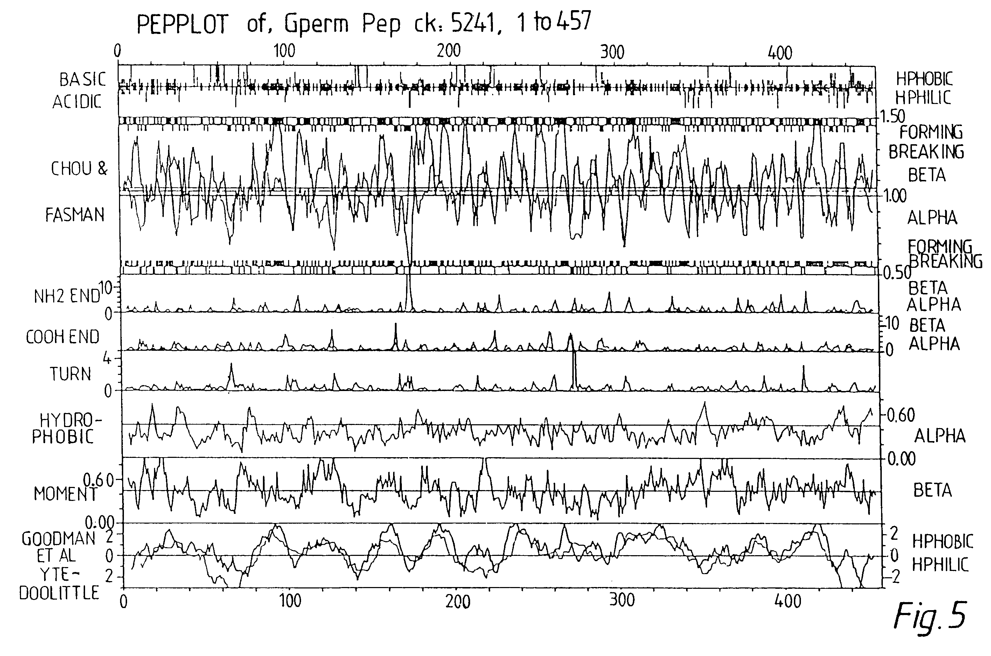

FIG. 5 is an analysis of the structure of the glucuronide permease using the University of Wisconsin Genetics Computer Group PepPlot program. The bottom panel shows the hydropathy plot generated using either the Goldman or the Kyte and Doolittle criteria. The many hydrophobic domains indicate potential alpha helical trans-membrane segments. This plot is very similar to a plot obtained analyzing the melibiose permease sequence.

### 5. DETAILED DESCRSIPTION OF THE INVENTION

The invention relates to the glucuronide permease gene, chimeric gene constructs and their expression products. Recombinant nucleotide vectors constructed to contain the glucuronide permease gene can be used to transform a variety of host cells. When these constructs are engineered to contain appropriate expression control elements, transformants will express glucuronide permease or a derivative encoded by the glucuronide permease gene.

The invention is based, in part, upon the discovery that the expression of glucuronide permease in this fashion alters the permeability of the host cell membrane to β-glucuronides, thus permitting the entrance of β-glucuronidase substrates. The method of the invention can provide for the detection of "reporter" β-glucuronidase in vivo, as well as for introducing metabolically active compounds into cells.

For purposes of clarity in description, and not by way of limitation, the invention will be described in three parts: (a) the glucuronide permease gene and expression product; (b) glucuronide permease chimeric genes and fusion proteins; and (c) use of glucuronide permease and glucuronide permease fusion protein.

### 5.1. THE GLUCURONIDE PERMEASE GENE AND PROTEIN

The glucuronide permease gene sequence and its deduced amino acid sequence are depicted in FIG. 3. These sequences, or their functional equivalents, can be used in accordance with the invention. For example, the sequences depicted in FIG. 3 can be altered by substitutions, additions or deletions that provide for functionally equivalent molecules. Due to the degeneracy of nucleotide coding sequences, other DNA sequences which encode substantially the same amino acid sequence as depicted in FIG. 3 may be used in the practice of the present invention. These include but are not limited to nucleotide sequences comprising all or portions of the glucuronide permease sequence depicted in FIG. 3 which are altered by the substitution of different codons that encode the same or a functionally equivalent amino acid residue within the sequence, thus producing a silent change. For example, one or more amino acid residues within the sequence can be substituted by another amino acid of a similar polarity which acts as a functional equivalent. Substitutes for an amino acid within the sequence may be selected from other members of the class to which the amino acid belongs. For example, the non-polar (hydrophobic) amino acids include alanine, leucine, isoleucine, valine, proline, phenylalanine, tryptophan and methionine. The polar neutral amino acids include glycine, serine, threonine, cysteine, tyrosine, asparagine, and glutamine. The positively charged (basic) amino acids include arginine, lysine, and histidine. The negatively charged (acidic) amino acids include aspartic and glutamic acid.

The glucuronide permease sequence was derived as described in the subsections below.

### 5.1.1. SEQUENCING THE GLUCURONIDE PERMEASE GENE

Upon sequencing the gene for β-glucuronidase (Jefferson et al., 1986, Proc. Natl. Acad. Sci. U.S.A. 83:8447-8451), sequence analysis indicated the presence of a second open reading frame of at least 340 bp, whose initiator codon overlapped the translational terminator of the β-glucuronidase gene. This open reading frame was found to be translationally active (Jefferson, R.A., 1985), Dissertation, University of Colorado, Boulder). Figure 2 illustrates the clones used to analyze the uid A locus of E. coli. pRAJ220 plasmid was used to deduce the sequence of β-glucuronidase; pRAJ210 was subcloned and the fragments 3' to the β-glucuronidase gene, encoding the open reading frame, were sequenced. The resulting DNA sequence and predicted protein are shown in FIG. 3

### 5.1.2. LOCATING THE GLUCURONIDE PERMEASE CODING REGION

By analogy with existing operons of E. coli, it was proposed that the open reading frame encoded a permease protein that could facilitate the uptake of β-glucuronides. The lactose and melibiose operons (for example) consist of a gene encoding a hydrolytic enzyme followed by a cocistronic gene for the corresponding transport protein, or permease. This format, of genes with interdependent functions being located on the same mRNA and subject to the same controlling mechanisms, is ubiquitous in bacteria. Because the substrates for glucuronidase are very polar, it is certain that they require active transport across the bacterial membrane. The level of genetic analysis performed on the uid locus would not have distinguished a mutation that eliminated β-glucuronidase function from a mutation that eliminated transport of a substrate, consistent with tight linkage between β-glucuronidase and glucuronide permease. In 1961, Francois Stoeber, in his Ph.D thesis in Paris, France, described the properties of a glucuronide permease in E. coli. His work clearly established the existence of such a transport mechanism, but did not in any way address the genetics or molecular biology of the system. These facts led to the hypothesis that the open reading frame encoded the glucuronide permease. Further analysis has also indicated that the range of substrates for β-glucuronidase that can be transported by the glucuronide permease is much wider than that of any previously described glycoside permease.

### 5.1.3 ANALYSIS OF AMINO ACID SEQUENCE AND THE GLUCURONIDE PERMEASE PROTEIN

The predicted amino acid sequence of the putative glucuronide permease was subjected to computer analysis to determine the existing sequences to which it had closest homology. The only two sequences that had significant homology to the glucuronide permease were the melB gene product, the melibiose permease, and the lacY gene product, the lactose permease. Of these, the homology with the meliblose permease is the strongest, and is shown in FIG. 4. Interestingly, both the melibiose and lactose permeases are members of a class of sugar transporters that use the proton gradient of the cell membrane to drive the transport of the sugar against a concentration gradient. These permeases are also in the unusual class that have been purified and shown to be active as single proteins, and whose activity can readily be reconstituted in vitro by addition of the pure permease to membrane vesicles.

The deduced amino acid sequence for glucuronide permease was subjected to a computer analysis to predict the structure of the protein. Such results are shown in FIG. 5. The salient feature of the analysis is the extremely hydrophobic nature of the protein, and the long stretches of hydrophobic amino acids that could easily span a membrane. The Kyte-Doolittle predictions of hydropathy (shown in the bottom frame) reveal hydrophobic regions that are located at almost identical positions to those of the melibiose permease, and at very similar positions to those of the lactose permease.

As explained in the following subsections, glucuronide permease may be readily produced using recombinant DNA techniques. However, in accordance with the invention, the production of glucuronide permease is not limited to genetic engineering techniques. All or portions of the amino acid sequence depicted in FIG. 3, including alterations such as substitutions, additions or deletions that yield functionally equivalent molecules, could be produced by chemical synthetic techniques well known to those skilled in the art.

### 5.1.4. MOLECULAR GENETIC DEMONSTRATION OF GLUCURONIDE PERMEASE

The proof of the glucuronide permease function was obtained by cloning the putative permease under the control of a heterologous promoter, in this case the promoter of the lactose operon of E. coli Kl2. When wild-type E. coli cells are plated on LB agar petri plates containing the chromogenic substrate for GUS, 5-bromo-4-chloro-3-indolyl-beta-D-glucuronide (called X-Gluc), the colonies remain white. When excess glucuronidase is present in the cells, for instance when encoded by a plasmid, the colony turns blue, due to deposition of the indigo dye. The blue color in this case is caused by (GUS) enzyme that is released from the many broken cells in the colony. This tends to give a relatively diffuse blue colony, with dye being deposited on the agar around the colony as well as on the colony itself. If however, a plasmid containing, not GUS, but rather the permease gene linked to the lac promoter, is introduced into the wild-type cells, the colonies on X-gluc become deep blue. The phenotype is even more striking because the blue color is very discrete, and is strictly localized to the colony. These colonies do not produce any detectable GUS in the absence of X-gluc, but rather are induced to produce it when X-gluc is present. This is due to the X-gluc being transported into the cell, binding to the uidR (gus R) gene product (the repressor of the uid operon) and allowing expression of GUS. This phenomenon requires glucuronide permease action. This can best be seen in the series of cloning experiments summarized in Table 1.

The glucuronide permease gene was subcloned into pUC19 to give a gene fusion with lac that caused E. coli Kl2 to give discrete blue colonies on X-gluc. This was then subjected to various changes to alter the reading frame of the predicted permease to determine whether the reading frame was required for the blue colony phenotype. Restriction endonuclease sites were chosen that were distributed throughout the gene. Some of these are indicated in FIG. 3. The restriction sites were cleaved and filled in to mutate the area around the site by shifting the putative reading frame. Such a shift occurring upstream of the glucuronide permease initiator codon (pRAJ281) showed no change in the color of the resulting colony. However, frame shifts within the coding sequence eliminated or severely reduced the capacity of the gene to give rise to colored colonies. In particular the Nsi I site mutant (PRAJ282) was completely colorless and the Acc I site mutant (pRAJ283) was almost completely colorless, with just a trace of blue after two days. The Ban II site frame-shift showed a faint trace of blue overnight with an obvious, but still quite pale, blue after two days on plates. The elimination of all blue color by the Nsi I mutant is expected, as the amount of permease made before the frame shift is very small - on the order of 100 amino acids. The next frame shift, the Acc I mutant, showed a trace of permease action. This may be because the amount of permease made (more like half of the permease) could have residudual activity. The Ban II mutant (in which more than 80% of the permease is made) shows a definite but severely reduced activity. This is also as would be predicted, and demonstrates conclusively the role of the open reading frame in the development of the blue colony.

The deletion mutant that extended 3' from the Nco site at bp 1510 (pRAJ285) caused no obvious change, leaving dark blue colonies. This verified the 3' extent of the gene as predicted by DNA sequencing. The context of the start site of the gene was altered by oligonucleotide mutagenesis in order to verify its location. This resulted in a permease gene deleted of sequences up to -12 from the initiator codon. This mutant showed an even darker blue colony (and smaller - presumably due to the over-expression of the membrane protein). The higher level of permease in these cells may be due to better translation on the new mRNA, perhaps because of loss of attenuating sequences. This clone, pRAJ286, contains a Bam HI linker immediately 5' of the Shine/Delgarno sequence and ensures that the initiator codon is the first one presented on any hybrid mRNA produced from this cloned fragment. To make a more useful cassette, pRAJ286 was modified by the addition of another Bam HI linker at the 3' end. This vector, pRAJ287, contains the entire glucuronide permease gene as a Bam HI fragment within the polylinker sites of the plasmid pUC19.

Next, the ability of the glucuronide permease to transport substrates other than X-gluc was tested. If the permease could transport such a large heterocyclic molecule as X-gluc, it was reasonable that it could transport other complex glucuronides, and hence offer a general route to transporting GUS substrates. Two bacterial cultures, one containing the plasmid pRAJ230 and the other pRAJ210 (Jeffeson et al. 1986) were grown to similar densities in L broth. Both these cultures produce GUS within the cells. pRAJ210 also includes the DNA encoding the permease - pRAJ230 is deleted of most of the permease gene. The cultures were washed extensively to eliminate GUS from the medium, and incubated with a solution of 4-methyl-umbelliferyl glucuronide, a fluorogenic substrate for GUS. The culture containing pRAJ210 immediately began to fluoresce intensely, while the culture containing pRAJ230 did not. When the cultures were lysed with a sonicator in the presence of fluorogenic substrate, both extracts showed intense fluorescence indicative of intact β-glucuronidase activity.

### 5.2. USES OF GLUCURONIDE PERMEASE AND GLUCURONIDASE FUSION PROTEINS

The glucuronide permease gene may be used in many different organisms to transport substrates for β-glucuronidase (GUS) into cells. Because this permease activity is encoded by a single polypeptide, and because there is no subsequent modification of the permease required for its insertion into membranes or its function (by analogy with the melibiose and lactose permeases) it is reasonable to expect that expression of the permease under the control of virtually any promoter in a transgenic organism will result in the transport of β-glucuronides into the cells of that organism.

### 5.2.1. GLUCURONIDE PERMEASE USED TOGETHER WITH THE GUS GENE FUSION MARKER

In one embodiment of the present invention, the glucuronide permease gene can be transfected together with GUS as part of the same construct, or incorporated into another vector (i.e. cotransfector), such as a plasmid or a eukaryotic vector such as SV-40 (Mulligan and Berg, 1980, Science 209:1422-1427). This in turn will allow substrates for β-glucuronidase, including fluorogenic and colorimetric β-glucuronide substrates, to be incorporated into live, undisrupted cells, thus allowing detection of β-glucuronidase reporter gene activity in vivo, eliminating the constraints of tissue extracts and histologic procedures, and thereby providing for more general applicability of the GUS system.

### 5.2.2. GLUCURONIDE PERMEASE USED WITHOUT THE GUS GENE FUSION MARKER

In another embodiment of the present invention, the glucuronide permease gene can be introduced into cells which have endogenous β-glucuronidase activity. By altering the number of glucuronide permease molecules present in the cell membrane, the permeability of the membrane to β-glucuronides can be controlled, and thus, glucuronide permease itself can function as a reporter gene.

### 5.2.3. GLUCURONIDE PERMEASE USED WITH A VARIETY OF PROMOTERS

It has been shown, supra, that the glucuronide permease gene, as part of plasmid pRAJ210 and in its native position downstream from the β-glucuronidase gene, is actively transcribed and translated into a functional protein in bacteria. In other experiments, also descried supra, functional glucuronide permease was produced when the isolated glucuronide permease gene was controlled by the heterologous lac promotor. Knowledge of the nucleotide sequence of the glucuronide permease gene, together with a knowledge of restriction enzyme specificities, allows one skilled in the art to combine the glucuronide permease gene with a variety of promotor elements and thus enable tight control of permease expression in cells transformed with the glucuronide permease gene. For example, promoters of different transcriptional activities could be used to produce corresponding levels of glucuronide permease; tissue-specific promoters, or developmentally controlled promoters, for example, are types of promoters which could be used.

Promoters which might be used to control glucuronide permease expression provided for in the present invention include, but are not limited to, the SV40 early promoter region (Bernoist and Chambon, 1981, Nature 290:304-310), the promoter contained in the 3' long terminal repeat of Rous sarcoma virus (Yamamoto, et al., 1980, Cell 22:787-797), the herpes thymidine kinase promoter (Wagner et al., 1981, Proc. Natl. Acad. Sci. U.S.A. 78:144-1445), the regulatory sequences of the metallothionine gene (Brinster et al., 1982, Nature 296:39-42); prokaryotic expression vectors such as the β-lactamase promoter (Villa-Kamaroff, et al., 1978, Proc. Natl. Acad. Sci. U.S.A. 75:3727-3731), or the tac promoter (DeBoer, et al., 1983, proc. Natl. Acad. Sci. U.S.A. 80:21-25), see also "Useful proteins from recombinant bacteria" in Scientific American, 1980, 242:74-94; plant expression vectors comprising the nopaline synthetase promoter region (Herrera-Estrella et al., Nature 303:209-213) or the cauliflower mosaic virus 35S RNA promoter (Gardner, et al., 1981, Nucl. Acids Res. 9:2871), and the chloroplast promoter for the photosynthetic enzyme ribulose biphosphate carboxylase (Herrera-Estrella et al., 1984, Nature 310:115-120); and the following animal transcriptional control regions, which exhibit tissue specificity and have been utilized in transgenic animals: elastase I gene control region which is active in pancreatic acinar cells (Swift et al., 1984, Cell 38:639-646; Ornitz et al., 1986, Cold Spring Harbor Symp. Quant. Biol. 50:399-409; MacDonald, 1987, Hepatology 7:425-515); insulin gene control region which is active in pancreatic beta cells (Hanahan, 1985, Nature 315:115-122), immunoglobulin gene control region which is active in lymphoid cells (Grosschedl et al., 1984, Cell 38:647-658; Adames et al., 1985, Nature 318:533-538; Alexander et al., 1987, Mol. Cell. Biol. 7:1436-1444), mouse mammary tumor virus control region which is active in testicular, breast, lymphoid and mast cells (Leder et al., 1986, Cell 45:485-495), albumin gene control region which is active in liver (Pinkert et al., 1987, Genes and Devel. 1:268-276), alpha-fetoprotein gene control region which is active in liver (Krumlauf et al., 1985, Mol. Cell. Biol. 5:1639-1648; Hammer et al., 1987, Science 235:53-58); alpha 1-antitrypsin gene control region which is active in the liver (Kelsey et al, 1987, Genes and Devel. 1:161-171), beta-globin gene control region which is active in myeloid cells (Mogram et al., 1985, Nature 315:338-340; Kollias et al., 1986, Cell 46:89-94; myelin basic protein gene control region which is active in oligodendrocyte cells in the bran (Readhead et al., 1987, Cell 48:703-712); myosin light chain-2 gene control region which is active in skeletal muscle (Sani, 1985, Nature 314:283-286), and gonadotropic releasing hormone gene control region which is active in the hypothalamus (Mason et al., 1986, Science 234:1372-1378).

### 5.2.4. GLUCURONIDE PERMEASE DELIVERED TO SELECTED CELL TARGETS

Glucuronide permease can be produced in large amounts by inserting the gene into an active expression vector and allowing the gene to be expressed, for example, in bacteria. In one embodiment of the present invention, the glucuronide permease could be chemically or genetically linked to a ligand, which could deliver the permease for insertion into cell membranes bearing the ligand receptor. By analogy to lactose and melibiose permease, the glucuronide permease should be able to integrate spontaneously into the cell membrane. For example, glucuronide permease could be coupled to the Fc region of an immunoglobulin specific for a discrete population of mammalian cells. Upon binding to these cells, the antibody would deliver glucuronide permease for insertion into the cell membrane, thereby making a discrete population of mammalian cells increasingly permeable to β-glucuronides. In this example, selected β-glucuronides could then be used to various purposes (see infra), utilizing endogenous β-glucuronidase expressed by mammalian cells.

In another, related embodiment of the present invention, glucuronide permease could be incorporated into membrane vesicles, and thereby become inserted into the cell membrane. The membrane vesicles could contain various substances, including, but not limited to β-glucuronide conjugated compounds.

### 5.2.5. GLUCURONIDE PERMEASE USED TO FACILITATE THE UPTAKE OF β-GLUCURONIDE CONJUGATES

The present invention provides for the use of glucuronide permease in altering membrane permeability to various compounds, utilizing β-glucuronide conjugates and endogenous or exogenously-supplied β-glucuronidase activity. These compounds include, but are not limited to, the following substances which either may be conjugated to β-glucuronide or transported themselves by glucuronide permease.
(a) Indicator substances such as histochemical indicators including napthol and napthol ASBl; fluorogenic substances such as 4-methyl umbelliferone and fluoroscein 3-O-methylfluoroscein, and colorimetric indicators such as resorufin, p-nitrophenol, and phenolphthalein.
(b) catabolic substances such as cellobiuronic acid, a disaccharide which, when transported into the cell, is metabolized to glucose by β-glucuronidase.
(c) Growth factors, such as, the various peptide growth hormones, and in plants, cytokinin or auxin.
(d) Toxic substances, such as snake venom toxins, including, according to their mode of action, cardiotoxins which cause irreversible depolarization of the cell membranes of heart muscles or nerve cells, neurotoxins which prevent neuromuscular transmission by blocking neurotransmitter receptors, and enzyme inhibitors which inhibit acetylcholine esterase and similar enzymes involved in nerve transmission. Also included are phytotoxins such as ricin and abrin and bacterial toxins, herbicides such as dinitrophenol derivatives and chemotherapeutic agents.
(e) Various steroid hormones are excreted as conjugated β-glucuronides. Using glucuronide permease, these hormones could be recycled, and their mechanism of action potentiated in select cells. In another embodiment of the present invention, exogenous steroid-β-glucuronides could be targeted to glucuronide permease expressing cells.
(f) Antibiotics which are deactivated by glucuronyl transferase, such as chloramphenicol, could be recycled, and their bioactivity half-life thereby extended.

By increasing the permeability of the cell membrane to β-glucuronides in both directions, glucuronide permease could facilitate the export of toxic glucuronides. For example, glucuronide permease could accelerate the removal of bilirubin deposits from the nervous systems of infants born with erythrobiastosis fetalis, a severe condition, caused by massive hemolysis secondary to maternal anti-Rh antibodies, which can result in hemoglobin deposition and bilirubin accumulation in the neonatal brain, with subsequent mental retardation.

The present invention is not to be limited in scope by the genes and proteins exemplified which are intended as but single illustrations of one aspect of the invention. Indeed, various modifications of the invention in addition to those shown and described herein will become apparent to those skilled in the art from the foregoing description and accompanying figures. Such modifications are intended to fall within the scope of the appended claims.

Plasmids containing the GUS gene have been deposited in November 1986 with the National Collection of Industrial and Marine Bacteria (NCIMB) Torry Research Station, PO Box 31, 135 Abbey Road, Aberdeen, UK AB9 8DG, including those plasmids known as pBI101, including pBI101.1 (previously known as pTAK1), (NCIB accession No. 12353), pBI101.2 (previously known as pTAK2) (NCIB accession No. 12354), and pBI101.3 (previously known as pTAK3) (NCIB accession No. 12355).

**Table 1**

| Behaviour of E. coli strain DH5- containing various lacZ-glucuronide permease fusions in pUC19 | |
|---|---|
| Plasmid | Color on X-Gluc plates |
| pRAJ 280 (lacZ/permease fusion) | Blue |
| pRAJ 281 (Sst I lacZ frameshift) | Blue |
| pRAJ 282 (Nsi I frameshift) | White |
| pRAJ 283 (Acc I frameshift) | White (trace of blue at 2 days) |
| pRAJ 284 (Sst I & Ban II frameshift) | Whitish (very pale blue) |
| pRAJ 285 (Nco I - Pst I 3' deletion) | Blue |
| pRAJ 286 (5' end, Bam HI linker) | Dark Blue (small) |
| pRAJ 287 (3' end Bam HI linker) | Dark Blue (small) |

## Claims

1. A recombinant DNA molecule comprising a glucuronide permease gene under the transcriptional control of a heterologous promoter, in which the glucuronide permease gene (i) comprises the sequence substantially as depicted in Figure 3 from about nucleotide number 106 to about nucleotide number 1464 and (ii) encodes a molecule that has glucuronide permease activity.

2. A recombinant DNA molecule according to claim 1, in which the glucuronide permease gene further comprises the nucleotide sequence substantially as depicted in Figure 3 from about nucleotide number 94 to about nucleotide number 105.

3. A recombinant DNA molecule according to claim 1 or 2, in which the glucuronide permease gene is under the control of second nucleotide sequence that regulates gene expression so that glucuronide premease is expressed in a host transformed with the recombinant DNA molecules.

4. A host cell transformant containing a recombinant DNA molecule in accordance with claim 1, 2 or 3.

5. A host cell transformant according to claim 4, additionally containing a glucuronidase gene.

6. A host cell transformant according to claim 4 or 5, in which the host cell comprises an animal cell, plant cell or a bacterium.

7. A method of introducing a substrate for glucuronase into cells of an organism, comprising introducing to the organism a recombinant DNA molecule in accordance with any one of claims 1 to 3, not being a method of therapeutic treatment of the human or animal body.

8. A method of altering the permeability of live cells of an organism, comprising introducing to the organism a recombinant DNA molecule in accordance with any one of claims 1 to 3, not being a method of therapeutic treatment of the human or animal body.

## Patentansprüche

1. Rekombinantes DNA-Molekül, umfassend ein Glucuronidpermeasegen unter der Transkriptionskontrolle eines heterologen Promotors, wobei das Glucuronidpermeasegen (i) im wesentlichen die Sequenz gemäß Figur 3 etwa von Nucleotid Nr. 106 bis etwa Nucleotid Nr. 1464 umfaßt und (ii) ein Molekül codiert, das Glucuronidpermeaseaktivität aufweist.

2. Rekombinantes DNA-Molekül nach Anspruch 1, wobei das Glucuronidpermeasegen weiterhin im wesentlichen die Nucleotidsequenz gemäß Figur 3 etwa von Nucleotid Nr. 94 bis etwa Nucleotid Nr. 105 umfaßt.

3. Rekombinantes DNA-Molekül nach Anspruch 1 oder 2, wobei das Glucuronidpermeasegen unter der Kontrolle einer zweiten Nucleotidsequenz ist, die die Genexpression reguliert, so daß die Glucuronidpermease in einem Wirt exprimiert wird, der mit den rekombinanten DNA-Molekülen transformiert ist.

4. Wirtszell-Transformante, enthaltend ein rekombinantes DNA-Molekül gemäß Anspruch 1, 2 oder 3.

5. Wirtszell-Transformante nach Anspruch 4, die zusätzlich ein Glucuronidasegen enthält.

6. Wirtszell-Transformante nach Anspruch 4 oder 5, wobei die Wirtszelle eine tierische Zelle, eine Pflanzenzelle oder ein Bakterium umfaßt.

7. Verfahren zur Einführung eines Substrats für Glucuronidase in Zellen eines Organismus, umfassend die Einführung eines rekombinanten DNA-Moleküls nach einem der Ansprüche 1 bis 3 in den Organismus, wobei das Verfahren kein therapeutisches Verfahren am menschlichen oder tierischen Körper ist.

8. Verfahren zur Änderung der Permeabilität von lebenden Zellen eines Organismus, umfassend die Einführung eines rekombinanten DNA-Moleküls nach einem der Ansprüche 1 bis 3 in den Organismus, wobei das Verfahren kein therapeutisches Verfahren am menschlichen oder tierischen Körper ist.

## Revendications

1. Molécule d'ADN recombinant comprenant un gène de glucuronide perméase sous le contrôle transcriptionnel d'un promoteur hétérologue, caractérisée en ce que le gène de la glucuronide perméase (i) comprend la séquence essentiellement comme le montre la figure 3 à partir du nucléotide numéro 106 environ jusqu'au nucléotide numéro 1464 environ et (ii) code pour une molécule présentant une activité de glucuronide perméase.

2. Molécule d'ADN recombinant selon la revendication 1, caractérisée en ce que le gène de la glucuronide perméase comprend en outre la séquence nucléotidique essentiellement comme le montre la figure 3 à partir du nuclétotide numéro 94 environ jusqu'au nucléotide numéro 105 environ.

3. Molécule d'ADN recombinant selon la revendication 1 ou 2, caractérisée en ce que le gène de la glucuronide perméase est sous le contrôle d'une deuxième séquence nucléotidique régulant l'expression des gènes de telle sorte que la glucuronide perméase soit exprimée chez un hôte transformé par les molécules d'ADN recombinant.

4. Cellule hôte transformée contenant une molécule d'ADN recombinant selon la revendication 1, 2 ou 3.

5. Cellule hôte transformée selon la revendication 4, contenant en outre un gène de la glucuronidase.

6. Cellule hôte transformée selon la revendication 4 ou 5, caractérisée en ce que la cellule hôte comprend une cellule animale, une cellule végétale ou une bactérie.

7. Procédé d'introduction d'un substrat de la glucuronase dans les cellules d'un organisme, comprenant l'introduction dans l'organisme d'une molécule d'ADN recombinant selon l'une quelconque des revendications 1 à 3, caractérisé en ce cu'il ne s'agit pas d'un traitement thérapeutique du corps humain ou animal.

8. Procédé d'altération de la perméabilité des cellules vivantes d'un organisme, comprenant l'introduction dans l'organisme d'une molécule d'ADN recombinant selon l'une quelconque des revendications 1 à 3, caractérisé en ce qu'il ne s'agit pas d'un procédé de traitement thérapeutique du corps humain ou animal.
